Europäisches Patentamt

European Patent Office

Office européen des brevets

(11) Publication number: **0 495 116 B1**

## EUROPEAN PATENT SPECIFICATION

(45) Date of publication of patent specification: **18.10.95** (51) Int. Cl.[6]: **A61K 31/725**

(21) Application number: **91913123.5**

(22) Date of filing: **29.07.91**

(86) International application number:
**PCT/JP91/01013**

(87) International publication number:
**WO 92/02231 (20.02.92 92/05)**

(54) **ANTI-HUMAN IMMUNODEFICIENCY VIRUS AGENT.**

| | |
|---|---|
| (30) Priority: **02.08.90 JP 206300/90** | (73) Proprietor: **TAIHO PHARMACEUTICAL COMPANY LIMITED**<br>**1-27, Kanda Nishiki-cho, Chiyoda-ku**<br>**Tokyo 101 (JP)** |
| (43) Date of publication of application:<br>**22.07.92 Bulletin 92/30** | |
| (45) Publication of the grant of the patent:<br>**18.10.95 Bulletin 95/42** | Proprietor: **Taiho Fine Chemical Co., Ltd.**<br>**22, 200-banchi, Aza Toyohara, Oaza Motohara**<br>**Kamikawamachi, Kodama-gun**<br>**Saitama 376-02 (JP)** |
| (84) Designated Contracting States:<br>**AT BE CH DE DK ES FR GB GR IT LI LU NL SE** | |
| (56) References cited:<br>**WO-A-90/08784**<br>**WO-A-90/09181**<br>**JP-A- 6 310 601**<br>**JP-A- 6 345 223**<br>**JP-A-63 128 001** | (72) Inventor: **HOSHINO, Hiroo**<br>**1-14-5, Heiwamachi**<br>**Maebashi-shi,**<br>**Gunma 371 (JP)** |
| **CHEMICAL ABSTRACTS Abstract No. 109 (22): 196956c (Gekkan Yakuji, 29(6), 1167-1174 (1987) Kitakawa Isao: "Biologically active marine natural products").** | (74) Representative: **VOSSIUS & PARTNER**<br>**Postfach 86 07 67**<br>**D-81634 München (DE)** |

Rank Xerox (UK) Business Services
(3.10/3.09/3.3.3)

**CHEMICAL ABSTRACTS Abstract No. 97(22) :**
**1884lb (Zhonghyao Tongbao, 7(4),**
**27-29(1982) Fan, Huizeng; Chen, Judi: "A new**
**method for the isolation and purification of**
**an acidic mucopolysaccharide from**
**Stichopus japonicus")**

**CHEMICAL ABSTRACTS Abstract No. 95(16) :**
**138509r (Yao Hsueh T'ung Pao, 16(4),**
**58(1981) Chen, Chu-Ti; Fan, Huei-Tseng;**
**Wen, Yu-Lin: "Observations on the stability**
**of said mucopolysaccharide potassium salts**
**from Stichopus Japonicus")**

**CHEMICAL ABSTRACTS Abstract No. 94(8) :**
**52763m (Fan, Hui-Zeng; Chen, Ju-Di; Lin, Ke-**
**Zhong: "Isolation of mucopolysaccharide**
**from Stichopus japonicus selenka and some**
**of its physical and chemical properties.")**

**Description**

The present invention relates to a novel antiviral agent effective against the human immunodeficiency virus (referred to as HIV in the specification).

Acquired Immune Deficiency Syndrome (AIDS) is a serious immunodeficiency disease caused by HIV infection [Nature 321, 10 (1986)]. Because of the high mortality AIDS has become a great social problem. Therefore, a counter-measure to the HIV infection is highly desirable.

Azidothymidine (AZT), which has an inhibiting activity on reverse transcriptase, is known as an effective anti-HIV agent and is regarded as a clinically effective agent at present.

However, the clinical effect of azidothymidine (AZT) used as an anti-HIV agent is not satisfactory, in addition azidothymidine has the problem that strong side-effects are caused by AZT, for example damage of bone marrow (hematopoietic tissue), neurological complaints, such as headache, or convulsion. In particular, in case of HIV, its genome infiltrates a chromosome of an infected cell as a provirus, and the cell seems to exist in a condition of hereditary disease so that a long-term administration of a medicament such as AZT is inevitably required and the side-effects of AZT become a great difficulty when used as an anti-HIV agent.

Under the present condition, development of new pharmaceutical preparations having an effect on HIV infection and AIDS with small side-effects and being capable of long-term administration is needed.

The present inventors conducted extensive research on compounds having an excellent effect on HIV, and found that polyfucose sulfates from a sea cucumber and/or pharmaceutically acceptable salts thereof have an excellent anti-HIV activity and are safe when administerd to patients for a long term.

Thus, the present invention provides an anti-HIV agent containing a polyfucose sulfate from a sea cucumber and/or pharmaceutically acceptable salts thereof as an active ingredient.

The polyfucose sulfate from a sea cucumber used in the invention is a polyfucose sulfate (hereinafter referred to as PFS) extracted from the body wall of Holothurian and has the physicochemical properties shown below.

*   Characteristics: white, amorphous and highly hygroscopic powder
*   Molecular weight: about 20,000 to about 200,000 daltons (as measured by polyacrylamide gel electrophoresis)
*   Analysis of composition: the composition by weight is as follows:

| | |
|---|---|
| Hexosamine | 1 to 7 wt.% |
| Fucose | 45 to 65 wt.% |
| Sulfate | 25 to 42 wt.%. |

The following methods were used to analyze Hexosamine (hereinafter referred to as HexN), Fucose (hereinafter referred to as Fuc) and Sulfate.

*   HexN
    Blumenkrantz method [Clin. Biochem., 9: 269 (1976)]
*   Fuc
    Dische method [J. Biol. Chem., 175: 595 (1948)]
*   Sulfate
    Dodgson method [Biochem. J., 78: 312 (1961)]

PFS is a known substance, described in, for example Yaoxue Xuebao, 1983, 18(3), 203-208; Science 1969, 166: 758-759; J. Biol. Chem., 1973, 248(1), 30-33; J. Biochem., 1972, 72, 1265-1267; Hirosaki Igaku, 1956, 7, 142-149 respectively, and can be easily produced by the methods described therein.

More specifically, PFS can be produced by extracting a body wall of Holothurian which has been hydrolyzed by alkaline hydrolysis or enzymes, such as pancreatin, followed by separation and purification of the extracts. Examples of sea cucumbers useful in the preparation of PFS are:

Stichopus japonicus Selenka,
Stichopus chloronoyus Brandt,
Stichopus variegatus Semper,
Holothuria pervicax Selenka,
Holothuria atra,
Holothuria argus,
Holothuria edulis,
Holothuria scabra,

Parastichopus nigripunctatus,
Thelenota ananas,
Holothuria monacaria Lesson,
Holothuria leucospilota Brandt,
Cucumaria chronhjelmi,
Cucumaria echinata,
Cucumaria frondosa Japonica,
Pentacta australis,
Paracaudina chilensis ransonneti,
Molpadia musculus,
Leptosynapta inhaerens,
Polycheira rufescens,
Synapta maculata,
Halodeima cinerascens(Brandt),
Actinopyga lacanora(Jaeger),
Actinopyga echinites(Jaeger),
Microthele nobilis(Selenka).

The cucumber to be used as the starting material may be a raw or dried one. Of the cucumbers exemplified above, Stichopus japonicus Selenka is the most preferred starting material.

The above analytical results show that PFS has in the molecule a sulfate group which reacts with bases to form a salt. These polyfucose sulfates are stable in the form of a salt and are usually isolated and purified in the form of a sodium and/or potassium salt. These polyfucose sulfate salts can be converted into a free polyfucose sulfate by treatment with cation exchange resins such as Dowex 50W (product of Dow Chemical Co.). Further, they can be converted into various desired salts by conducting conventional salt exchange when desired. Usable salts of polyfucose sulfate are pharmaceutically acceptable salts including salts of potassium, sodium or like alkali metals, and salts of calcium, magnesium, barium or like alkaline earth metals, or pyridinium salt or like organic bases. The method for preparing PFS will be described below in detail in the following reference examples.

The polyfucose sulfates and/or pharmaceutically acceptable salts thereof are formulated into various pharmaceutical compositions useful for HIV treatment. Stated more specifically, the anti-HIV agents of the present invention comprising an effective amount of the active ingredient of the invention and a pharmaceutically acceptable carrier can be prepared in various administration forms in accordance with various conditions of HIV treatment. The administration form can be any of tablets, capsules, powders, granules, grains, solutions, emulsions, suspensions and like oral forms, and injections, suppositories, semisolids, plasters and like parenteral forms. These preparations can be manufactured by conventional methods already known to those skilled in the art. A solid preparation for oral administration can be prepared by mixing the effective component of the invention with an excipient with or without addition of binders, disintegrators, lubricants, coloring agents, flavorings, perfumes, etc. and making them into tablets, capsules, powders, granules, grains or the like in a conventional manner. Injection preparations can be produced by adding a pH-adjusting agent, buffer, stabilizer, isotonizing agent, localanesthetic and the like to the effective component of the invention, and converting the mixture into intravenous, intramuscular, subcutaneous, intracutaneous or intraperitoneal injections in a conventional manner. Suppositories can be prepared by formulating a mixture of the effective component, base materials and optionally a surfactant and the like into a suppository in a conventional manner. A semisolid preparation can be prepared by adding base materials, a pH-adjusting agent, a lubrication-auxiliary agent, optionally adding antiseptics, preservatives and the like when required, to the effective component of the invention and formulating the mixture into ointment, cream, jelly and the like in a conventional manner, for use as a lubricant.

Examples of excipients useful for oral solid preparations are lactose, sucrose, starch, talc, magnesium stearate, crystalline cellulose, methyl cellulose, carboxymethyl cellulose, glycerin, sodium arginate or gum arabic. Examples of binders useful for oral preparations include polyvinyl alcohol, polyvinyl ether, ethyl cellulose, gum arabic, shellac and sucrose. Examples of useful lubricants are magnesium stearate or talc. The coloring agents, disintegrators and other auxiliaries to be added include those commonly used in the art. Tablets may be coated by well-known methods.

Examples of base materials useful for suppositories include oily base materials such as macrogol, lanolin, cacao oil, fatty acid triglyceride and Witepsol® (registered trademark for the product of Dynamite-Nobel AG).

Examples of base materials useful for semisolid preparations include carboxymethyl cellulose, sodium carboxymethyl cellulose and similar gelating agents. Examples of pH-adjusting agents useful for semisolid

4

preparations include sodium hydroxide and aqueous ammonia. Examples of useful lubrication-auxiliary agents are polyethylene glycol, polyvinyl alcohol, carboxymethyl cellulose, sodium alginate or polyacrylic acid. Examples of useful antiseptics and preservatives are paraoxybenzoate esters (methyl, ethyl, propyl and butyl ester).

The amount of the effective component per each unit dosage varies with the symptoms of the patient to be given the preparation, the form of the preparation, etc. Usually a preferred amount is 10 to 200 mg in an oral preparation, 1 to 100 mg in a injection and 10 to 100 mg in a suppository, per each unit dosage. The daily clinical dosage of the composition of the invention also varies with the patient's age, sex, conditions and other factors but usually may be in the range of about 10 to about 1,000 mg, preferably about 50 to about 200 mg in terms of the effective component per day and can be given at 1 to 4 divided doses.

As a lubricant, the present agent can be used for prevention of HIV infection by applying the agent to the vulva or the penis before a sexual act.

The present invention will be described in greater detail with reference to Reference Examples, Examples and Pharmacological Tests. The percentages in the Reference Examples and Examples are all by weight.

## REFERENCE EXAMPLE 1

Preparation of PFS-1

Starting material was prepared by pounding 4 kg of fresh Stichopus japonicus Selenka whose viscus had been removed after washing with water, followed by dehydration with ethanol treatment to give dried powder. To a predetermined quantity of the powder was added 50 times as much water as the powder by weight, and the mixture was stirred overnight at room temperature while maintaining the pH of the solution at about 8 by adding diluted sodium hydroxide solution. The insoluble material was removed by centrifugation and filtration. The removed insoluble material was further extracted two or three times under the same conditions as above. The obtained extracts were combined and dialyzed against water, and a formed precipitate was filtrated. A 1 % benzidine hydrochloride solution was added to the filtrate until no further precipitate was formed. The formed precipitate was collected and washed with 0.1 % benzidine hydrochloride solution. The precipitate was suspended in water and stirred for 2 hours while maintaining the pH of the solution at 8 by adding diluted sodium hydroxide solution to remove benzidine which was separated from the solution. After the filtrate was neutralized and concentrated, a small quantity of potassium acetate and ethanol 4 times the volume of the residue was added to the residue. A formed precipitate was collected by centrifugation, washed with ethanol and ether in this sequence and dried to give 7.9 g of a crude product.

A 500 mg portion of this crude product was dissolved in 50 ml of 0.02 M sodium phosphate buffer (pH 6.5) containing 1 M sodium chloride and purified on a DEAE-Cellulose (product of Sigma Chemical Co.) column (2.5 x 50 cm). Elution was conducted by using as an eluant 0.02 M sodium phosphate buffer (pH 6.5) containing sodium chloride whereby the concentration of sodium chloride was increased from 1M to 3M at a flow rate of 30 ml/h. The fractions whose sodium chloride concentration was 2 M were collected, dialysed against water and lyophilized to give 47 mg of extract from 500 mg of the crude product. 100 mg of the extract prepared by repeating the above procedure were dissolved in 10 ml 0.02 M sodium phosphate buffer (pH 6.5) containing 1 M sodium chloride and purified by the same procedure as above. The fractions whose sodium chloride concentration was 2 M were collected, dialyzed against water and lyophilized to give 47 mg of extract from 100 mg. This extract was dissolved in 5 ml 0.02 M sodium phosphate buffer (pH 6.5) containing 1 M sodium chloride and purified on a DEAE-Cellulose column (1.4 x 27 cm). The elution was conducted in conditions using as an eluant 0.02 M sodium phosphate buffer (pH 6.5) containing sodium chloride whereby the concentration of sodium chloride was increased from 1M to 2M at a flow rate of 10 ml/h. The fractions whose sodium chloride concentration was 1.5 M were collected, dialyzed against water and lyophilized to give 28 mg of purified product from 47 mg. The analytical values of the purified product are as follows.

Molecular weight: about 170,000 to about 180,000 daltons (as determined by polyacrylamide gel electrophoresis)

Fucose: 59.5 %

Sulfate: 32.7 %

Hexosamine: 1.83 %

Uronic acid: 0.13 %

Pentose: 1.04 %

Protein: 1.00 %

Ash: 22.2 %.

REFERENCE EXAMPLE 2

Preparation of PFS-2

Dried Stichopus japonicus Selenka was pulverized, and lipids were removed with acetone. 2 kg of this dried and delipidized Stichopus japonicus Selenka were put into 13.6 l of 0.2 M potassium acetate solution. Subsequently 16 g of Protin A (product of Daiwa Fine Chemical Co.), which is a protease, were added to the mixture and the resulting mixture was stirred at 70 °C for 1 hour. 15 g of Protin A was added again to the solution and the resulting mixture was stirred at 70 °C for 2.5 hours. After removing insoluble material by centrifugation, 320 g of sodium chloride were added, and then ethanol was added until the concentration of ethanol became 20 %, and the mixture was allowed to stand overnight to remove a precipitate formed by centrifugation. Saturated cetylpyridinium chloride solution was added to the supernatant until no further precipitate was formed, the mixture being allowed to stand overnight to collect a precipitate formed and the collected precipitate was washed with water. This precipitate was dissolved in 1680 ml of ethanol under heating and the solution was cooled to room temperature to collect a precipitate formed. This precipitate was dissolved in 2 l of n-propanol under heating and the solution was cooled to room temperature to collect a precipitate formed. This precipitate was dissolved in 1600 ml of n-propanol : water (2:1) solution under heating and the solution was cooled to room temperature. The precipitate formed was removed by centrifugation, and to the supernatant was added 1 % (W/V) of potassium acetate to collect a precipitate formed. This precipitate was washed with ethanol and acetone in this sequence, and dried under reduced pressure to give 34.2 g of a crude product. This crude product was dissolved in 855 ml of 1M sodium hydroxide solution containing 2 % of sodium chloride. To this solution were added dropwise 85.5 ml of hydrogen peroxide solution (30 %), and the mixture was stirred at 60 °C for 6 hours. The solution was cooled to room temperature, and the insoluble material formed was removed by centrifugation. The pH of the solution was adjusted to 6.5. To the solution was added ethanol until the concentration of ethanol became 35 %, and the mixture was cooled to 4 °C. The precipitate formed was removed by centrifugation, and ethanol was further added to the supernatant until the concentration of ethanol became 56 %. The mixture was cooled to 4 °C to collect a precipitate formed by centrifugation. The precipitate was dissolved in 220 ml of water, and the solution was added dropwise to 1100 ml of ethanol containing 0.5 % of potassium acetate. The mixture was cooled to 4 °C to collect a precipitate formed by centrifugation. The precipitate was washed with ethanol and acetone in this sequence, and dried under reduced pressure to give 24.1 g of purified product. The analytical values of the purified product are as follows.

Molecular weight: about 100,000 to about 110,000 daltons (as determined by polyacrylamide gel electrophoresis)

$[\alpha]_D^{20}$ : -199.78 °

Fucose: 53.6 %

Hexosamine: 5.1 %

Uronic acid: 0.4 %

Sulfate: 35.6 %

Protein: 1.7 %

Sodium: 5.7 %

Potassium: 6.4 %.

REFERENCE EXAMPLE 3

Preparation of PFS-3

5.3 g of PFS having the following physicochemical characteristics were produced by the same procedure as described in Reference Example 2.

Molecular weight: about 120,000 to about 130,000 daltons (as determined by polyacrylamide gel electrophoresis method)

$[\alpha]_D^{20}$ : -204.23 °

Fucose: 53.3 %

Hexosamine: 4.2 %

Uronic acid: 0.3 %

Sulfate: 32.6 %

Protein: 0.8 %
Sodium: 5.0 %
Potassium: 9.6 %.

REFERENCE EXAMPLE 4

Preparation of PFS-4

The dried body wall of Holothuria leucospilota Brandt was cut into pieces, hydrolyzed in potassium hydroxide solution, and the mixture was neutralized with acetic acid. The mixture was filtrated and concentrated. The concentrate was allowed to stand in a refrigerator overnight to give a transparent supernatant solution after centrifugation. Ethanol was added to the solution in an amount which was twice the volume of the solution to give a precipitate. The precipitate was dissolved in a proper amount of water, and the solution was subjected to oxidized decolorization. To the decolorized solution ethanol was added again which was twice the volume of the solution to give a crude product. The crude product was dissolved in 2 M potassium chloride solution in an amount of 1:30 (W/V), and the solution was allowed to stand in a refrigerator overnight. After centrifugation the precipitate was removed. To the transparent supernatent solution was added the same volume of hexadecylmethylammonium bromide solution, and the precipitate was decolorized by washing and precipitation with 1 M potassium chloride using Celite-545 (product of Wako Pure Chemical Ind.) as a filtration auxiliary and subsequently washed with 1.5 M of sodium chloride to conduct dissociation. A small quantity of the solution was dispensed and ethanol was added to the solution to give a precipitate. The precipitate was subjected to an agarose electrophoresis (hexadiamine buffer) to give a dissociated solution. Ethanol was added to the solution to give a precipitate. After hydrolyzing the precipitate, ethanol was added again to give a precipitate. The precipitate was washed with ethanol and acetone, dehydrated and dried to give purified product. The analytical values of the purified product are as follows.

Molecular weight: about 84,000 daltons (as determined by polyacrylamide gel electrophoresis)
$[\alpha]_D^{20}$ : -151.3°
Fucose: 51.0 %
Hexosamine: 2.7 %
Sulfate: 29.3 %.

Each polyfucose sulfate obtained in the above Reference Examples 1 to 4 showed a single spot on electrophoresis [Dietrich C. P., J. Chromatogr., 130, 299 (1977)].

EXAMPLE 1

Injection

PFS sodium potassium salt produced in Reference Example 2 was dissolved in water for injection to give a 5 % water solution. This solution was packed in a vial for lyophilization in an amount corresponding to 50 mg of a polyfucose sulfate from Stichopus japonicus Selenka per one vial, and lyophilization was conducted. 2 ml of physiological saline for dissolution were added to the vial separately.

EXAMPLE 2

Injection

An injection preparation was prepared according to the following prescription.

| | |
|---|---|
| PFS sodium potassium salt (Reference Example 2) physiological saline | 40 mg proper quantity |
| per 1 sample | 2 ml |

EXAMPLE 3

Tablet

A tablet was prepared according to the following prescription.

| PFS sodium potassium salt (Reference Example 2) | 10 mg |
| cornstarch | 65 mg |
| carboxymethyl cellulose | 20 mg |
| polyvinylpyrrolidone | 3 mg |
| magnesium stearate | 2 mg |
| per 1 tablet | 100 mg |

EXAMPLE 4

Suppository

A suppository was prepared according to the following prescription.

| PFS sodium potassium salt (Reference Example 2) | 50 mg |
| Witepsol W-35 (product of Dynamite-Nobel AG) | 950 mg |
| per 1 piece | 1000 mg |

EXAMPLE 5

Lubricant (aqueous lubricating jelly)

| | W/V % |
| --- | --- |
| PFS sodium potassium salt (Reference Example 2) | 0.3-0.5 |
| carboxyvinyl polymer | 0.1 |
| polyethylene glycol | 10.0 |
| 1 N-sodium hydroxide | proper quantity |
| perfume | proper quantity |
| purified water | proper quantity |
| | total 100 ml |

Polyethylene glycol and PFS sodium potassium salt were dissolved and stirred in 10 ml of purified water in this sequence, and then carboxyvinylpolymer was dispersed in the solution. Thereafter the pH of the solution was adjusted to 6.3 using 1N sodium hydroxide and purified water was added to the solution to give a total volume of 100 ml. Then a perfume was added and the solution was treated in an autoclave at 100 °C for 20 minutes.

PHARMACOLOGICAL TEST

(1) Anti-HIV Activity

MT-4 cells [Miyoshi I. et al., Gann Monogr., 28, 219-228 (1982)] were adjusted to provide a $2 \times 10^5$ cells/ml medium (RPMI-1640 + 10 % FCS). To 500 $\mu$l of the medium were added 50 $\mu$l of a solution of sea cucumber-derived polyfucose sulfate obtained in Reference Example 2 in varying concentrations. Two hours after the addition, the cells were infected by HIV in a proportion of $5 \times 10^3$ Pfu/well. Four days later, a smear of MT-4 cells was formed, and HIV antigen positive cells were determined by fluorescent antibody technique [Harada S. et al., Science, 229, 563-566, (1985); Takeuchi, Y. et al, Jpn. J. Cancer Res.(Gann), 78, 11-15 (1987)].

The proportion of virus-infected cells (HIV antigen positive cells) is shown in Table 1 below.

Table 1

| PFS concentration of Reference Example 2 (µg/ml) | 10 | 1 | 0 reference |
|---|---|---|---|
| Infected cell proportion (%) | 0 | 10 | 95 |

The above results show that the anti-HIV agent of the invention inhibits infection by HIV effectively.

(2) cytotoxicity

The cytotoxicity of polyfucose sulfate from sea cucumber was determined in accordance with the above (1). MT-4 cells were prepared in a concentration of $1 \times 10^5$ cells/ml and cultured for 4 days in the presence of sea cucumber-derived polyfucose sulfate obtained in Reference Example 2 in varying concentrations. Then the number of the cells was counted and the cell survival ratios in each concentration were calculated with the cell survival ratio of the reference cells being 100 %, and the cytotoxicity was evaluated. The results are shown in Table 2 below.

Table 2

| PFS concentration of Reference Example 2 (µg/ml) | 100 | 10 | 1 | 0 reference |
|---|---|---|---|---|
| Surviving cell proportion (%) | 105 | 105 | 104 | 100 |

The above results show that the anti-HIV agent of the invention has no significant cytotoxicity.

INDUSTRIAL APPLICABILITY

The anti-HIV agent of the present invention has an effect on the prevention of HIV infection, the prevention of a crisis of AIDS and AIDS related complex [ARC; R.R.Redfield & D.S.Burke, Science, 18, 74-87, (1988)] and the therapy thereof and can be suitably used in a long-term administration because of the low cytotoxicity of the active ingredient, i.e., polyfucose sulfate from sea cucumber.

Therefore, the anti-HIV agent of the present invention has an effect on the prevention of HIV infection to uninfected people, the prevention of a crisis of a carrier and the therapy thereof and is very effective to cure a patient of AIDS and ARC.

**Claims**

1. An anti-HIV agent containing a polyfucose sulfate from sea cucumber having the following physicochemical properties and/or a pharmaceutically acceptable salt thereof as an active ingredient:
   Characteristics; white, amorphous and highly hygroscopic powder
   Molecular weight: about 20,000 to about 200,000 daltons (as measured by polyacrylamide gel electrophoresis)
   Composition by weight:
   Hexosamine 1 to 7 wt.%
   Fucose 45 to 65 wt.%
   Sulfate 25 to 42 wt.%.

2. An anti-HIV agent according to claim 1 containing a polyfucose sulfate from sea cucumber having the following physicochemical properties and/or a pharmaceutically acceptable salt thereof as an active ingredient:
   Molecular weight: about 170,000 to about 180,000 daltons (as measured by polyacrylamide gel electrophoresis)

Composition by weight:
Hexosamine: 1.83 wt.%
Fucose: 59.5 wt.%
Sulfate: 32.7 wt.%
Uronic acid: 0.13 wt.%
Pentose: 1.04 wt.%
Protein: 1.00 wt.%
Ash: 22.2 wt.%.

3. An anti-HIV agent according to claim 1 containing a polyfucose sulfate from sea cucumber having the following physicochemical properties and/or a pharmaceutically acceptable salt thereof as an active ingredient:
Molecular weight: about 100,000 to about 110,000 daltons (as measured by polyacrylamide gel electrophoresis)
$[\alpha]_D^{20}$ : -199.78 °
Composition by weight:
Hexosamine: 5.1 wt.%
Fucose: 53.6 wt.%
Sulfate: 35.6 wt.%
Uronic acid: 0.4 wt.%
Protein: 1.7 wt.%
Sodium: 5.7 wt.%
Potassium: 6.4 wt.%.

4. An anti-HIV agent according to claim 1 containing a polyfucose sulfate from sea cucumber having the following physicochemical properties and/or a pharmaceutically acceptable salt thereof as an active ingredient:
Molecular weight: about 120,000 to about 130,000 daltons (as measured by polyacrylamide gel electrophoresis)
$[\alpha]_D^{20}$ : -204.23 °
Composition by weight:
Hexosamine: 4.2 wt.%
Fucose: 53.3 wt.%
Sulfate: 32.6 wt.%
Uronic acid: 0.3 wt.%
Protein: 0.8 wt.%
Sodium: 5.0 wt.%
Potassium: 9.6 wt.%.

5. An anti-HIV agent according to claim 1 containing a polyfucose sulfate from sea cucumber having the following physicochemical properties and/or a pharmaceutically acceptable salt thereof as an active ingredient:
Molecular weight: about 84,000 daltons (as measured by polyacrylamide gel electrophoresis)
$[\alpha]_D^{20}$ : -151.3 °
Composition by weight:
Hexosamine: 2.7 wt.%
Fucose: 51.0 wt.%
Sulfate: 29.3 wt.%.

6. Use of a polyfucose sulfate from sea cucumber and/or a pharmaceutically acceptable salt thereof for the preparation of a pharmacological composition for treating diseases caused by HIV, wherein the polyfucose sulfate has the following physicochemical properties:
Characteristics: white, amorphous and highly hygroscopic powder
Molecular weight; about 20,000 to about 200,000 daltons (as measured by polyacrylamide gel electrophoresis)
Composition by weight:
Hexosamine 1 to 7 wt.%
Fucose 45 to 65 wt.%

10

EP 0 495 116 B1

Sulfate 25 to 42 wt.%.

7. Use according to claim 6 wherein said polyfucose sulfate and/or the pharmaceutically acceptable salt thereof has the following physicochemical properties:

Molecular weight; about 170,000 to about 180,000 daltons (as measured by polyacrylamide gel electrophoresis)
Composition by weight:
Hexosamine: 1.83 wt.%
Fucose: 59.5 wt.%
Sulfate: 32.7 wt.%
Uronic acid: 0.13 wt.%
Pentose: 1.04 wt.%
Protein: 1.00 wt.%
Ash: 22.2 wt.%.

8. Use according to claim 6 wherein said polyfucose sulfate and/or the pharmaceutically acceptable salt thereof has the following physicochemical properties;

Molecular weight: about 100,000 to about 110,000 daltons (as measured by polyacrylamide gel electrophoresis)
$[\alpha]_D^{20}$ : -199.78°
Composition by weight:
Hexosamine: 5.1 wt.%
Fucose: 53.6 wt.%
Sulfate: 35.6 wt.%
Uronic acid: 0.4 wt.%
Protein: 1.7 wt.%
Sodium: 5.7 wt.%
Potassium: 6.4 wt.%.

9. Use according to claim 6 wherein said polyfucose sulfate and/or the pharmaceutically acceptable salt thereof has the following physicochemical properties:

Molecular weight; about 120,000 to about 130,000 daltons (as measured by polyacrylamide gel electrophoresis)
$[\alpha]_D^{20}$ : -204.23°
Composition by weight:
Hexosamine: 4.2 wt.%
Fucose: 53.3 wt.%
Sulfate: 32.6 wt.%
Uronic acid: 0.3 wt.%
Protein: 0.8 wt.%
Sodium: 5.0 wt.%
Potassium: 9.6 wt.%.

10. Use according to claim 6 wherein said polyfucose sulfate and/or the pharmaceutically acceptable salt thereof has the following physicochemical properties:

Molecular weight: about 84,000 daltons (as measured by polyacrylamide gel electrophoresis)
$[\alpha]_D^{20}$ : -151.3°
Composition by weight:
Hexosamine: 2.7 wt.%
Fucose: 51.0 wt.%
Sulfate: 29.3 wt.%.

**Patentansprüche**

1. Anti-HIV-Mittel, das als Wirkstoff ein Polyfucosesulfat aus Seegurken mit den folgenden physikalisch-chemischen Eigenschaften und/oder ein pharmazeutisch verträgliches Salz davon enthält:
Merkmale: weißes, amorphes und stark hygroskopisches Pulver,
Molekulargewicht: etwa 20 000 bis etwa 200 000 Dalton (gemessen durch Polyacrylamidgelelektro-

11

phorese),

Zusammensetzung (bezogen auf das Gewicht):

Hexosamin 1 bis 7 Gew.-%

Fucose 45 bis 65 Gew.-%

Sulfat 25 bis 42 Gew.-%.

2. Anti-HIV-Mittel nach Anspruch 1, das als Wirkstoff ein Polyfucosesulfat aus Seegurken mit den folgenden physikalisch-chemischen Eigenschaften und/oder ein pharmazeutisch verträgliches Salz davon enthält:

Molekulargewicht: etwa 170 000 bis etwa 180 000 Dalton (gemessen durch Polyacrylamidgelelektrophorese),

Zusammensetzung (bezogen auf das Gewicht):

Hexosamin 1,83 Gew.-%

Fucose 59,5 Gew.-%

Sulfat 32,7 Gew.-%

Uronsäure 0,13 Gew.-%

Pentose 1,04 Gew.-%

Protein 1,00 Gew.-%

Asche 22,2 Gew.-%.

3. Anti-HIV-Mittel nach Anspruch 1, das als Wirkstoff ein Polyfucosesulfat aus Seegurken mit den folgenden physikalisch-chemischen Eigenschaften und/oder ein pharmazeutisch verträgliches Salz davon enthält:

Molekulargewicht: etwa 100 000 bis etwa 110 000 Dalton (gemessen durch Polyacrylamidgelelektrophorese),

$[\alpha]_D^{20}$ : -199,78°,

Zusammensetzung (bezogen auf das Gewicht):

Hexosamin 5,1 Gew.-%

Fucose 53,6 Gew.-%

Sulfat 35,6 Gew.-%

Uronsäure 0,4 Gew.-%

Protein 1,7 Gew.-%

Natrium 5,7 Gew.-%

Kalium 6,4 Gew.-%

4. Anti-HIV-Mittel nach Anspruch 1, das als Wirkstoff ein Polyfucosesulfat aus Seegurken mit den folgenden physikalisch-chemischen Eigenschaften und/oder ein pharmazeutisch verträgliches Salz davon enthält:

Molekulargewicht: etwa 120 000 bis etwa 130 000 Dalton (gemessen durch Polyacrylamidgelelektrophorese),

$[\alpha]_D^{20}$ : -204,23°,

Zusammensetzung (bezogen auf das Gewicht):

Hexosamin 4,2 Gew.-%

Fucose 53,3 Gew.-%

Sulfat 32,6 Gew.-%

Uronsäure 0,3 Gew.-%

Protein 0,8 Gew.-%

Natrium 5,0 Gew.-%

Kalium 9,6 Gew.-%,

5. Anti-HIV-Mittel nach Anspruch 1, das als Wirkstoff ein Polyfucosesulfat aus Seegurken mit den folgenden physikalisch-chemischen Eigenschaften und/oder ein pharmazeutisch verträgliches Salz davon enthält:

Molekulargewicht: etwa 84 000 Dalton (gemessen durch Polyacrylamidgelelektrophorese),

$[\alpha]_D^{20}$ : -151,3°,

Zusammensetzung (bezogen auf das Gewicht):

Hexosamin 2,7 Gew.-%

Fucose 51,0 Gew.-%

Sulfat 29,3 Gew.-%.

6. Verwendung eines Polyfucosesulfats aus Seegurken mit den folgenden physikalisch-chemischen Eigenschaften und/oder eines pharmazeutisch verträglichen Salzes davon zur Herstellung eines Arzneimittels für die Behandlung von Krankheiten, die durch HIV verursacht werden, wobei das Polyfucosesulfat die folgenden physikalisch-chemischen Eigenschaften aufweist:

Merkmale: weißes, amorphes und stark hygroskopisches Pulver,
Molekulargewicht: etwa 20 000 bis etwa 200 000 Dalton (gemessen durch Polyacrylamidgelelektrophorese),
Zusammensetzung (bezogen auf das Gewicht):
Hexosamin 1 bis 7 Gew.-%
Fucose 45 bis 65 Gew.-%
Sulfat 25 bis 42 Gew.-%,

7. Verwendung nach Anspruch 6, wobei das Polyfucosesulfat und/oder das pharmazeutisch verträgliche Salz davon die folgenden physikalisch-chemischen Eigenschaften aufweist:

Molekulargewicht: etwa 170 000 bis etwa 180 000 Dalton (gemessen durch Polyacrylamidgelelektrophorese),
Zusammensetzung (bezogen auf das Gewicht):
Hexosamin 1,83 Gew.-%
Fucose 59,5 Gew.-%
Sulfat 32,7 Gew.-%
Uronsäure 0,13 Gew.-%
Pentose 1,04 Gew.-%
Protein 1,00 Gew.-%
Asche 22,2 Gew.-%.

8. Verwendung nach Anspruch 6, wobei das Polyfucosesulfat und/oder das pharmazeutisch verträgliche Salz davon die folgenden physikalisch-chemischen Eigenschaften aufweist:

Molekulargewicht: etwa 100 000 bis etwa 110 000 Dalton (gemessen durch Polyacrylamidgelelektrophorese),
$[\alpha]_D^{20}$ : -199,78°,
Zusammensetzung (bezogen auf das Gewicht):
Hexosamin 5,1 Gew.-%
Fucose 53,6 Gew.-%
Sulfat 35,6 Gew.-%
Uronsäure 0,4 Gew.-%
Protein 1,7 Gew.-%
Natrium 5,7 Gew.-%
Kalium 6,4 Gew.-%.

9. Verwendung nach Anspruch 6, wobei das Polyfucosesulfat und/oder das pharmazeutisch verträgliche Salz davon die folgenden physikalisch-chemischen Eigenschaften aufweist:

Molekulargewicht: etwa 120 000 bis etwa 130 000 Dalton (gemessen durch Polyacrylamidgelelektrophorese),
$[\alpha]_D^{20}$ : -204,23°,
Zusammensetzung (bezogen auf das Gewicht):
Hexosamin 4,2 Gew.-%
Fucose 53,3 Gew.-%
Sulfat 32,6 Gew.-%
Uronsäure 0,3 Gew.-%
Protein 0,8 Gew.-%
Natrium 5,0 Gew.-%
Kalium 9,6 Gew.-%.

10. Verwendung nach Anspruch 6, wobei das Polyfucosesulfat und/oder das pharmazeutisch verträgliche Salz davon die folgenden physikalisch-chemischen Eigenschaften aufweist:

Molekulargewicht: etwa 84 000 Dalton (gemessen durch Polyacrylamidgelelektrophorese),

$[\alpha]_D^{20}$ : -151,3°,
Zusammensetzung (bezogen auf das Gewicht):
Hexosamin 2,7 Gew.-%
Fucose 51,0 Gew.-%
Sulfat 29,3 Gew.-%.

## Revendications

1.  Agent anti-VIH contenant un sulfate de polyfucose provenant d'holothurie ayant les propriétés physico-chimiques suivantes et/ou un sel de celui-ci acceptable du point de vue pharmaceutique en tant que composant actif:
    caractéristiques: poudre blanche, amorphe et hautement hygroscopique,
    poids moléculaire: environ 20.000 à environ 200.000 daltons (tel que mesuré par électrophorèse sur gel de polyacrylamide)
    composition en poids:
    hexosamine 1 à 7% en poids
    fucose 45 à 65% en poids
    sulfate 25 à 42% en poids.

2.  Agent anti-VIH suivant la revendication 1, contenant un sulfate de polyfucose provenant d'holothurie ayant les propriétés physico-chimiques suivantes et/ou un sel de celui-ci acceptable du point de vue pharmaceutique en tant que composant actif:
    poids moléculaire: environ 170.000 à environ 180.000 daltons (tel que mesuré par électrophorèse sur gel de polyacrylamide)
    composition en poids:
    hexosamine 1,83% en poids
    fucose 59,5% en poids
    sulfate 32,7% en poids
    acide uronique 0,13% en poids
    pentose 1,04% en poids
    protéine 1,00% en poids
    cendres 22,2% en poids.

3.  Agent anti-VIH suivant la revendication 1, contenant un sulfate de polyfucose provenant d'holothurie ayant les propriétés physico-chimiques suivantes et/ou un sel de celui-ci acceptable du point de vue pharmaceutique en tant que composant actif:
    poids moléculaire: environ 100.000 à environ 110.000 daltons (tel que mesuré par électrophorèse sur gel de polyacrylamide)
    $[\alpha]^{20}_D$: -199,78°
    composition en poids:
    hexosamine 5,1% en poids
    fucose 53,6% en poids
    sulfate 35,6% en poids
    acide uronique 0,4% en poids
    protéine 1,7% en poids
    sodium 5,7% en poids
    potassium 6,4% en poids.

4.  Agent anti-VIH suivant la revendication 1, contenant contenant un sulfate de polyfucose provenant d'holothurie ayant les propriétés physico-chimiques suivantes et/ou un sel de celui-ci acceptable du point de vue pharmaceutique en tant que composant actif:
    poids moléculaire: environ 120.000 à environ 130.000 daltons (tel que mesuré par électrophorèse sur gel de polyacrylamide)
    $[\alpha]^{20}_D$: -204,23°
    composition en poids:
    hexosamine 4,2% en poids
    fucose 53,3% en poids
    sulfate 32,6% en poids

acide uronique 0,3% en poids
protéine 0,8% en poids
sodium 5,0% en poids
potassium 9,6% en poids.

5. Agent anti-VIH suivant la revendication 1, contenant un sulfate de polyfucose provenant d'holothurie ayant les propriétés physico-chimiques suivantes et/ou un sel de celui-ci acceptable du point de vue pharmaceutique en tant que composant actif:
   poids moléculaire: environ 84.000 daltons (tel que mesuré par électrophorèse sur gel de polyacrylamide)
   $[\alpha]^{20}_D$: -151,3 °
   composition en poids:
   hexosamine 2,7% en poids
   fucose 51,0% en poids
   sulfate 29,3% en poids.

6. Utilisation d'un sulfate de polyfucose provenant d'holothurie et/ou d'un sel de celui-ci acceptable du point de vue pharmaceutique pour la préparation d'une composition pharmacologique pour le traitement de maladies provoquées par VIH, dans laquelle le sulfate de polyfucose a les propriétés physico-chimiques suivantes:
   caractéristiques: poudre blanche, amorphe et hautement hygroscopique,
   poids moléculaire: environ 20.000 à environ 200.000 daltons (tel que mesuré par électrophorèse sur gel de polyacrylamide)
   composition en poids:
   hexosamine 1 à 7% en poids
   fucose 45 à 65% en poids
   sulfate 25 à 42% en poids.

7. Utilisation suivant la revendication 6, dans laquelle ce sulfate de polyfucose et/ou le sel de celui-ci acceptable du point de vue pharmaceutique a les propriétés physico-chimiques suivantes :
   poids moléculaire: environ 170.000 à environ 180.000 daltons (tel que mesuré par électrophorèse sur gel de polyacrylamide)
   composition en poids:
   hexosamine 1,83% en poids
   fucose 59,5% en poids
   sulfate 32,7% en poids
   acide uronique 0,13% en poids
   pentose 1,04% en poids
   protéine 1,00% en poids
   cendres 22,2% en poids.

8. Utilisation suivant la revendication 6, dans laquelle ce sulfate de polyfucose et/ou le sel de celui-ci acceptable du point de vue pharmaceutique a les propriétés physico-chimiques suivantes :
   poids moléculaire: environ 100.000 à environ 110.000 daltons (tel que mesuré par électrophorèse sur gel de polyacrylamide)
   $[\alpha]^{20}_D$: -199,78 °
   composition en poids:
   hexosamine 5,1% en poids
   fucose 53,6% en poids
   sulfate 35,6% en poids
   acide uronique 0,4% en poids
   protéine 1,7% en poids
   sodium 5,7% en poids
   potassium 6,4% en poids.

9. Utilisation suivant la revendication 6, dans laquelle ce sulfate de polyfucose et/ou le sel de celui-ci acceptable du point de vue pharmaceutique a les propriétés physico-chimiques suivantes :
   poids moléculaire: environ 120.000 à environ 130.000 daltons (tel que mesuré par électrophorèse sur gel de polyacrylamide)

$[\alpha]^{20}_{D}$: -204,23 °

composition en poids:

hexosamine 4,2% en poids

fucose 53,3% en poids

sulfate 32,6% en poids

acide uronique 0,3% en poids

protéine 0,8% en poids

sodium 5,0% en poids

potassium 9,6% en poids.

10. Utilisation suivant la revendication 6, dans laquelle ce sulfate de polyfucose et/ou le sel de celui-ci acceptable du point de vue pharmaceutique a les propriétés physico-chimiques suivantes :

poids moléculaire: environ 84.000 daltons (tel que mesuré par électrophorèse sur gel de polyacrylamide)

$[\alpha]^{20}_{D}$: -151,3 °

composition en poids:

hexosamine 2,7% en poids

fucose 51,0% en poids

sulfate 29,3% en poids.